(19) Europäisches Patentamt
European Patent Office
Office européen des brevets

(11) Veröffentlichungsnummer: **0 199 066**
**A1**

# (12) EUROPÄISCHE PATENTANMELDUNG

(21) Anmeldenummer: **86103447.8**

(22) Anmeldetag: **14.03.86**

(51) Int. Cl.[4]: **C 07 C 1/26**
C 07 C 17/00, C 07 B 35/06

(30) Priorität: **20.03.85 DE 3510034**

(43) Veröffentlichungstag der Anmeldung:
**29.10.86 Patentblatt 86/44**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Dockner, Toni, Dr.**
**Grossgasse 6**
**D-6701 Meckenheim(DE)**

(72) Erfinder: **Krug, Herbert**
**Mussbacher Strasse 49**
**D-6700 Ludwigshafen(DE)**

(72) Erfinder: **Sann, Werner**
**Pfaustrasse 9**
**D-6700 Ludwigshafen(DE)**

## (54) Verfahren zur reduktiven Dehalogenierung von Halogenverbindungen.

(57) Reduktive Dehalogenierung von aliphatischen, cycloaliphatischen, aromatischen und araliphatischen Halogenverbindungen, bei denen mindestens ein Halogenatom kovalent an Kohlenstoff gebunden ist, indem man die Halogenierungen in der Gasphase im wesentlichen bei Normaldruck oder Überdruck mit Kohlenwasserstoffen an aktivem Kohlenstoff unter Bildung von Halogenwasserstoff umsetzt.

EP 0 199 066 A1

Croydon Printing Company Ltd

Verfahren zur reduktiven Dehalogenierung von Halogenverbindungen

Die Erfindung betrifft die reduktive Dehalogenierung von aliphatischen, cycloaliphatischen, aromatischen oder araliphatischen Halogenverbindungen, bei denen mindestens ein Halogenatom kovalent am Kohlenstoff gebunden ist.

Es ist bekannt, die reduzierende Eliminierung von vicinalen Dihalogeniden mit Metallen wie Na, K, Mg, Zn, Cu mit Organometallverbindungen oder mit Metallhydriden durchzuführen. Bei leichter eliminierbaren Halogenverbindungen können auch Halogenide, z.B. Iodide verwendet werden. Eine Zusammenfassung dieser Methoden findet sich in Houben-Weyl, Methoden der Organischen Chemie, 4. Aufl., Bd. V/1b, 1972. Die Reduktion vicinaler Bromchloride oder Dichloride mittels Natriumiodid ist in J. Org. Chem. 41, 1976, S. 3284 beschrieben.

Reagenzien wie Cr(II)-Salze, Tributylzinnhydrid oder $Me_3SnNa$ sind in J. Amer. Chem. Soc. 1968, S. 1582 bis 1589 und 1970, S. 2849 bis 2856 sowie in J. Org. Chem. 44, 1976, S. 4774 bis 4781 für die Elminierung vicinaler Dihalogenide verwendet worden.

Die hydrierende Dehalogenierung aliphatischer Halogenverbindungen mit Raney-Nickel in Gegenwart einer zum Halogen äquivalenten Menge an Alkali ist in Chem.Ber. 92, 1700 (1959) beschrieben worden. R. Baltzly und A.P. Phillips lehren die katalytische Hydrogenolyse von aromatischen Halogenverbindungen (J.Amer.Chem.Soc. 68, 261-265, 1946) in neutraler oder saurer wäßriger oder alkoholischer Lösung, wobei als Katalysator Edelmetalle wie Palladium oder Platin, z.B. Palladium/Tierkohle oder Adams-Katalysator Verwendung finden. Aliphatische Halogenverbindungen wurden unter den angegebenen Bedingungen nur dann reduziert, wenn das Halogen z.B. durch Doppelbindungen aktiviert ist. Nach G.E. Ham und W.P. Coker lassen sich in Vinyl- oder Allylstellung halogensubstituierte Olefine in Gegenwart von Rhodium, Palladium oder Platin zu Halogenalkanen hydrieren. Die Ausbeuten sind abhängig vom Lösungsmittel, Katalysatorträger und Vorhandensein von Thiophen (J.Org.Chem. 29, 194-198, 1964).

Die Entfernung aromatisch gebundenen Halogens und Ersatz durch Wasserstoff gelingt ebenfalls durch Metalle wie Na, K oder metallorganische Verbindungen oder auch Hydride (siehe Houben-Weyl, Methoden d. org. Chem., Bd. 5/4, S. 769, 1960). Darüber hinaus läßt sich Halogen in der aromatischen Reihe durch katalytisch angeregten Wasserstoff ersetzen. Als Katalysatoren werden Palladium und Raney-Nickel vorgeschlagen. Ein photochemisches Verfahren zur Reduktion halogensubstituierter aromatischer

Verbindungen ist in Tetrahedron Letters 16, 1267-1270 (1969) veröffentlicht worden.

In der JP-OS 78855/1977 (Anmeldenr. 156510/1975) wird ein Verfahren zur Herstellung von Biphenyl beschrieben, indem man Polychlorbiphenyl im Wasserstoffstrom über einem Metall wie Ni, Pd, Zn, Mg etc. pyrolisiert.

Nachteilig an den beschriebenen Verfahren zur reduktiven Dehalogenierung ist, daß sie technisch nicht oder nur eingeschränkt zu nutzen sind, da die Reagenzien sehr teuer sind und große Salzmengen, in vielen Fällen Schwermetallsalze anfallen.

Die japanische Offenlegungsschrift 216 075/1983 (Anmeldenr. 098 672/1982) lehrt, daß sich Polychlorbiphenyl auf Aktivkohle bei 400 bis 600°C zersetzt, wobei sich HCl und Kohlenwasserstoffe bilden. Der Grad der Enthalogenierung wurde durch $AgNO_3$-Titration bestimmt. Danach ergibt sich z.B. daß bei 400°C mehr als 4 Minuten für die 100 %ige Zersetzung von Biphenylhexachlorid benötigt werden, während bei 600°C bereits 10 Sekunden ausreichen. Angaben über die Art der entstehenden Kohlenwasserstoffe fehlen ebenso wie Ausbeuteangaben. In den Beispielen wird das eingesetzte Polychlorbiphenyl zwar in Hexan gelöst, eine Beteiligung des Lösungsmittels an der Umsetzung kann jedoch ausgeschlossen werden, da Hexan unter den angegebenen Reaktionsbedingungen vollständig verdampft wird. Bei den verwendeten hohen Reaktionstemperaturen und einem durch eine Saugpumpe erzeugten Unterdruck ist davon auszugehen, daß Hexan auch nicht adsorbiert an der Aktivkohle vorliegt.

Der Erfindung lag nun die Aufgabe zugrunde, ein Dehalogierungsverfahren zu finden, das sich technisch leicht durchführen läßt, billig ist, bei dem keine Schwermetallsalze anfallen und nach dem eine Vielzahl von Halogenverbindungen mit guter Ausbeute umgesetzt werden kann, wobei das ursprüngliche Kohlenstoffgerüst nicht zerstört wird.

Demgemäß wurde ein Verfahren zur reduktiven Dehalogenierung von aliphatischen, cycloaliphatischen, aromatischen und araliphatischen Halogenverbindungen, bei denen mindestens ein Halogenatom kovalent am Kohlenstoff gebunden ist, gefunden, das sich vorteilhaft in der Weise durchführen läßt, daß man die Halogenverbindungen in der Gasphase im wesentlichen bei Normaldruck oder Überdruck mit Kohlenwasserstoffen an aktivem Kohlenstoff unter Bildung von Halogenwasserstoff umsetzt.

Die Dehalogenierung kann in der Gasphase bei Reaktionstemperaturen von 200 bis 600, vorzugsweise 300 bis 500, insbesondere 350 bis 450°C vorgenommen werden. In der Regel verwendet man daher Kohlenwasserstoffe mit einem Siedepunkt unterhalb von 200°C. Die Struktur der reduzierenden Kohlenwasserstoffe ist in keiner Weise eingeschränkt. Bevorzugt wird man billige und niedrigsiedende aliphatische Kohlenwasserstoffe wie Methan, Propan, Isobutan, Cyclohexan, Naphtha, $C_4$- oder $C_5$-Schnitt einsetzen.

Als Kohlenstoff verwendet man z.B. Aktivkohle, wie Supersorbon® oder Contarbon®, zweckmäßigerweise in Form von Tabletten oder Strängen oder als Wirbelkontakt mit Kohlenstoffpartikelchen unterhalb von 1 mm. Es ist auch möglich, den aktiven Kohlenstoff im Umsetzungsgemisch zu erzeugen, indem man die Umsetzung in Gegenwart einer oberflächenaktiven festen Phase durchführt, wodurch vom ersten Augenblick der Reaktion an hieran katalytisch wirksamer Kohlenstoff abgeschieden wird.

Als feste Phase werden vorteilhaft oberflächenaktive Mineralien wie Kieselgel, Aluminiumoxid usw. verwendet. Beispielsweise kommen dazu auch die Oxide der Elemente der Hauptgruppe II, III, IV und/oder V des Periodensystems sowie der Nebengruppe IV in Betracht, wie MgO, $MgSiO_3$, CaO, $B_2O_3$, $TiO_2$, insbesondere Oxide des Siliziums oder Aluminiums.

Die Mengenverhältnisse von Kohlenwasserstoff zu Halogenverbindung liegen in der Regel bei 0,2 bis 5, insbesondere 0,5 bis 3. An aktivem Kohlenstoff sind katalytische Mengen ausreichend.

Als Halogenverbindungen werden erfindungsgemäß solche Verbindungen verwendet, die mindestens ein Halogenatom wie Iod, Brom oder Chlor tragen. Als aliphatische Verbindungen können gesättigte oder ungesättigte, offenkettige oder cyclische Verbindungen umgesetzt werden. Weiterhin können araliphatische und aromatische Halogenverbindungen gespalten werden.

Die reduktive Eliminierung vicinaler Dihalogenide oder die Spaltung von Monohalogeniden werden durch folgende Gleichung beschrieben:

a) $$R-CH(X)-CH(X)-R' + C_nH_{2n+2} \longrightarrow R-CH=CH-R' + 2\ HX + C_nH_{2n}$$

R, R' = H, Alkyl, Cycloalkyl, Aryl, Aralkyl
X = Cl, Br, I

b) $$2\ R-X + C_nH_{2n+2} \longrightarrow 2\ R-H + 2\ HX + C_nH_{2n}$$

R = Alkyl, Cycloalkyl, Aryl, Aralkyl
X = Cl, Br, I

Der für die Bildung von Halogenwasserstoff benötigte Wasserstoff entstammt dem Kohlenwasserstoff, aus dem neben wasserstoffärmeren Derivaten häufig Kohlenstoff als Endprodukt gebildet wird.

Für die erfindungsgemäße Umsetzung in Betracht kommende Ausgangsstoffe sind z.B. unverzweigte, verzweigte oder cyclische Monohalogenalkane wie Chlorethan, 1-Chlor-2-phenyl-ethan, 1-Chlorpropan, 1-Chlor-2-methylpropan, 2-Brompropan, 1-Iodbutan, 1-Chlorpentan, tert.-Butyliodid, Chlorcyclohexan oder -cyclopentan. Diese Verbindungen werden über die entsprechenden Alkene in die Alkane überführt, wobei häufig Gemische beider Produkte anfallen.

Weiterhin können olefinisch ungesättigte Monohalogenverbindungen zu Alkenen dehalogeniert werden, z.B. 1-Chlorprop-1-en, 2-Chlor-prop-1-en, Allylchlorid, 1-Brombut-1-en, 2-Brombut-2-en, 1-Iodpent-2-en, 1-Chlorcyclohex-1-en, 1-Bromcyclohex-2-en, Cinnamylchlorid oder $\beta$-Bromstyrol.

Besonders geeignete Ausgangsstoffe sind vicinale Dihalogenide der allgemeinen Formel I

$$R-\underset{X}{\underset{|}{CH}}-\underset{X}{\underset{|}{CH}}-R' \qquad I,$$

in der X für Iod, Brom und/oder Chlor steht und die Reste R und R' unabhängig voneinander Wasserstoff, einen aliphatischen Rest, z.B. eine Alkylgruppe mit 1 bis 20, insbesondere 1 bis 5 Kohlenstoffatomen, eine Cycloalkylgruppe, z.B. mit 5 bis 8 Kohlenstoffatomen, eine Arylgruppe oder eine Aralkylgruppe, insbesondere mit 6 bis 12 Kohlenstoffatomen bedeuten oder beide Reste zusammen mit den C-Atomen an die sie gebunden sind zu einem Cycloalkan mit 4 bis 12, insbesondere 4 bis 8 Kohlenstoffatomen verbunden sind. Weiterhin können vorteilhaft Polyhalogenverbindungen umgesetzt werden.

Beispielsweise kommen Verbindungen wie 1,2-Dichlorpropan, 1,2-Dichlorbutan, 1,2-Dibrombutan, 2,3-Diiodbutan, 1,2-Dibrom-2-methylpropan, 1,2-Dibromcyclobutan oder Cyclohexan, 1,2-Dichlor-1,2-diphenylethan, 1,2-Dichlor-3-phenyl-propan, 1-Brom-2-Iod-butan, 1-Brom-2-chlorethan in Betracht, sowie 1,1,2-Trichlorethan oder Hexachlorcyclohexan (HCH).

Durch die beschriebenen Ausgangsverbindungen soll die Anwendungsbreite des erfindungsgemäßen Verfahrens nicht eingeschränkt werden. Die Halogenverbindungen können zusätzlich unter den Reaktionsbedingungen inerte

Substituenten wie Cyano-, Alkoxy-, Dialkylamino-, Phenyl- oder substituierte Phenylgruppen tragen. Weiterhin können heterocyclische Halogenverbindungen umgesetzt werden.

Als aromatische Halogenverbindungen werden z.B. Halogenbezole wie Brombenzol, meta-Dichlorbenzol, 1,3,5-Trichlorbenzol, Halogentoluole wie p-Bromtoluol, 2,4-Dichlortoluol, halogenierte Biphenyle oder Triphenyle wie Diphenyl- oder Triphenylchlorid oder Polychlorbiphenyl (PCB) sowie halogenierte Naphthaline wie Chlor- oder Bromnaphthalin, Penta- oder Hexachlornaphthalin umgesetzt. Weiterhin kommen heteroaromatische Verbindungen, z.B. halogensubstituiertes Pyridin, Chinolin oder Imidazol in Betracht.

Die aromatischen Halogenverbindungen werden durch Wasserstoff substituiert. Mehrfach halogenhaltige Aromaten werden stufenweise reduktiv bis zum halogenfreien Kohlenwasserstoff enthalogeniert.

Es ist auch möglich, die erfindungsgemaß umzusetzenden Halogenverbindungen in Abwesenheit von Kohlenwasserstoffen als H-Donatoren zu Halogenwasserstoff und halogenfreien Verbindungen an Aktivkohle zu spalten. In diesem Fall fungiert die Halogenverbindung selbst als H-Donator, wodurch die Ausbeuten dementsprechend niedriger als bei Verwendung von Kohlenwasserstoffen ausfallen.

Die erfindungsgemäße Umsetzung kann diskontinuierlich oder kontinuierlich bei erhöhtem oder leicht vermindertem Druck z.B. bei 0,5 bis 100, insbesondere 0,1 bis 50, vorzugsweise 1 bis 10 bar nach den dafür üblichen Techniken durchgeführt werden. Besonders bevorzugt ist die Umsetzung bei Normaldruck.

Vorteilhaft wird die Umsetzung kontinuierlich durchgeführt, indem man wie der Zeichnung zu entnehmen ist, die Halogenverbindung (1) und den Kohlenwasserstoff (2) ggf. in einem Vorverdampfer (3) verdampft und gasformig über eine auf Reaktionstemperatur befindliche Kohlenschicht leitet. Der Kohlenstoff ist zweckmäßigerweise in Form von Tabletten oder Strängen in einer Festbettschicht in einem rohrenförmigen Reaktor (4) untergebracht. Die den Reaktor verlassenden Reaktionsgase werden dann uber eine Waschkolonne (5) geleitet, um den Halogenwasserstoff durch Wasserwäsche abzutrennen. Anschließend werden die als Abgase (6) anfallenden dehalogenierten Reaktionsprodukte durch Kondensation isoliert und gegebenenfalls z.B. destillativ gereinigt.

Nach dem erfindungsgemäßen Verfahren ist es überraschend auf billige und technisch einfache Art möglich, die Umwelt belastende toxische halogenierte Kohlenwasserstoffe in weniger giftige bzw. unbedenkliche Verbindungen zu überführen, wodurch Entsorgungsprobleme dieser z.T. in großen Mengen bei technischen Prozessen verwendeten bzw. anfallenden Stoffe gelöst werden, ohne daß die bei der Verbrennung üblichen Probleme auftreten. Über die Toxikologie halogenierter Kohlenwasserstoffe, insbesondere Chlorkohlenwasserstoffe siehe Ullmann's Enzyklopädie der techn. Chemie, 4. Aufl., Bd. 9, Seiten 481 bis 490, 521, 522 und 536.

Die nachfolgenden Beispiele erläutern die Erfindung.

Beispiel 1

Wie der abgebildeten Apparatur zu entnehmen ist, werden in einen Festbettofen (4) (L = 500 mm, D = 25 mm), der mit 70 g Aktivkohle (Contarbon BA® spezial, Körnung 4 mm) gefüllt ist, stündlich über einen Vorverdampfer (3) 15 ml 1,2-Dichlorpropan (1) mit 15 l iso-Butan (2) bei 350°C vorverdampft und bei 400°C über den Reaktor geleitet.

Die den Reaktor verlassenden Reaktionsgase werden in eine Waschkolonne (5) geleitet und von HCl befreit. Pro Stunde fallen 410 g wäßrige HCl mit 2,66 Gew.% HCl an. Dies entspricht einem Umsatz von 98 %. Man erhält pro Stunde 17,6 l Abgas (6) mit 16,85 Vol.% Propen und 0,1 Vol.% Propan neben kleineren Mengen Methan, Ethan, Ethen und Buten. Dies entspricht einer Ausbeute von 88,2 % an Propen, bezogen auf den Umsatz.

Vergleichsbeispiel 1a

Man verfährt wie in Beispiel 1, setzt aber statt 15 l iso-Buten 15 l Stickstoff ein. Pro Stunde fallen 410 g wäßrige HCl mit 2,66 Gew.% HCl an. Dies entspricht einem Umsatz von 98 %.

Man erhält pro Stunde 16,5 l Abgas mit 7,95 Vol.% Propen. Dies entspricht einer Ausbeute von 39 %, bezogen auf umgesetztes 1,2-Dichlorpropan.

Beispiel 2

Man verfährt wie in Beispiel 1, setzt aber statt 15 l iso-Butan 16 ml Cyclohexan ein. Pro Stunde fallen 410 g wäßrige HCl mit 2,67 Gew.% HCl an. Dies entspricht einem Umsatz von 98,2 %.

Daneben erhält man 17,6 l Abgas mit 17,3 Vol.% Propan. Dies entspricht einer Ausbeute von 91 %, bezogen auf den Umsatz.

Beispiel 3

Man verfährt wie in Beispiel 1, setzt aber statt 15 l iso-Butan 15 l Ethan ein. Pro Stunde fallen 410 g wäßrige HCl mit 2,66 Gew.% HCl an. Dies entspricht einem Umsatz von 98 %.

Man erhält 17,4 l Abgas mit 14,15 Vol.% Propen und 0,6 Vol.% Propan. Dies entspricht einer Ausbeute von 73,5 % an Propen und 3 % Propan, bezogen auf den Umsatz.

Beispiel 4

Man verfährt wie in Beispiel 1, setzt aber statt 15 l iso-Butan 15 l Methan ein. Pro Stunde fallen 410 g wäßrige HCl mit 2,66 Gew.% HCl an. Dies entspricht einem Umsatz von 98 %. Man erhält 17,2 l Abgas mit 10,85 Vol.% Propen und 0,4 Vol.% Propan.

Dies entspricht einer Ausbeute von 55,7 % an Propen und 2 % Propan bezogen auf den Umsatz.

Beispiel 5

Wie in Beispiel 1 beschrieben, werden 9 l Ethylchlorid und 15 l iso-Butan vorerhitzt und bei 430°C über den Reaktor geleitet. Man erhält 390 g wäßrige HCl mit 1,24 Gew.% HCl. Dies entspricht einem Umsatz von 33 %. An Abgas erhält man 18,1 l mit 10,13 Vol.% Ethan und 3,92 Vol.% Ethen.

Dies entspricht einer Ausbeute von 62 % Ethan und 24 % Ethen, bezogen auf den Umsatz.

Beispiel 6

Man verfährt wie in Beispiel 1, setzt aber statt 70 g Aktivkohle 200 g BASF-Silica-Perlen ein. Pro Stunde fallen 400 g wäßrige HCL mit 1,31 Gew.% HCl an. Dies entspricht einem Umsatz von 47 %.

Man erhält pro Stunde 16,5 l Abgas mit 4,0 Vol.% Propen. Dies entspricht einer Ausbeute von 41 %, bezogen auf den Umsatz.

Beispiel 7

Man verfährt wie in Beispiel 3, setzt aber 17 ml 1,2-Dichlorbenzol und 15 l iso-Butan ein. Man erhält 400 g wäßrige HCl mit 1,18 Gew.% HCl. Dies entspricht 43 % des eingesetzten Chlor. Daneben fallen 17 g Kondensat mit 20,2 Gew.% Benzol, 27,1 Gew.% Chlorbenzol und 51,8 Gew.% 1,2-Dichlorbenzol an. Dies entspricht einem Umsatz von 56 %.

Die Ausbeute, bezogen auf den Umsatz, betrug 48,9 % an Benzol und 45,5 % an Chlorbenzol.

Patentansprüche

1. Verfahren zur reduktiven Dehalogenierung von aliphatischen, cycloaliphatischen, aromatischen und araliphatischen Halogenverbindungen, bei denen mindestens ein Halogenatom kovalent an Kohlenstoff gebunden ist, dadurch gekennzeichnet, daß man die Halogenverbindungen in der Gasphase im wesentlichen bei Normaldruck oder Überdruck mit Kohlenwasserstoffen an aktivem Kohlenstoff unter Bildung von Halogenwasserstoff umsetzt.

2. Verfahren nach Anspruch 1, dadurch gekennzeichnet, daß man die Umsetzung an Aktivkohle durchführt.

3. Verfahren nach Anspruch 1, dadurch gekennzeichnet, daß man den aktiven Kohlenstoff erzeugt, indem man die Umsetzung in Gegenwart einer oberflächenaktiven festen Phase durchführt.

4. Verfahren nach Anspruch 3, dadurch gekennzeichnet, daß man als feste Phase Mineralien mit großer Oberfläche verwendet.

5. Verfahren nach Anspruch 3, dadurch gekennzeichnet, daß man als feste Phase Oxide der Elemente der Hauptgruppe II, III, IV und/oder V sowie der Nebengruppe IV des Periodensystems verwendet.

6. Verfahren nach Anspruch 1, dadurch gekennzeichnet, daß man niedrig siedende aliphatische Kohlenwasserstoffe verwendet.

7. Verfahren nach Anspruch 1, dadurch gekennzeichnet, daß man als Halogenverbindungen unverzweigte, verzweigte oder cyclische Monohalogenalkane umsetzt.

8. Verfahren nach Anspruch 1, dadurch gekennzeichnet, daß man olefinisch ungesättigte Monohalogenverbindungen umsetzt.

9. Verfahren nach Anspruch 1, dadurch gekennzeichnet, daß man vicinale Di- oder Polyhalogenide umsetzt.

10. Verfahren nach Anspruch 1, dadurch gekennzeichnet, daß man aromatische Halogenverbindungen wie Halogenbenzole oder Polychlorbiphenyl umsetzt.

11. Verfahren nach Anspruch 1, dadurch gekennzeichnet, daß man heterocyclische Halogenverbindungen umsetzt.

12. Verfahren nach Anspruch 1, dadurch gekennzeichnet, daß man Halogenverbindungen umsetzt, die als Halogenatome Chlor, Brom und/oder Iod enthalten.

13. Verfahren gemäß Anspruch 1, dadurch gekennzeichnet, daß man die Reaktion bei Temperaturen von 200 bis 600°C durchführt.

14. Verfahren gemäß Anspruch 1, dadurch gekennzeichnet, daß man die Reaktion bei einem Druck von 0,5 bis 100 bar durchführt.

15. Verfahren gemäß Anspruch 1, dadurch gekennzeichnet, daß man die Reaktion bei einem Druck von 1 bis 10 bar durchführt.

Zeichn.

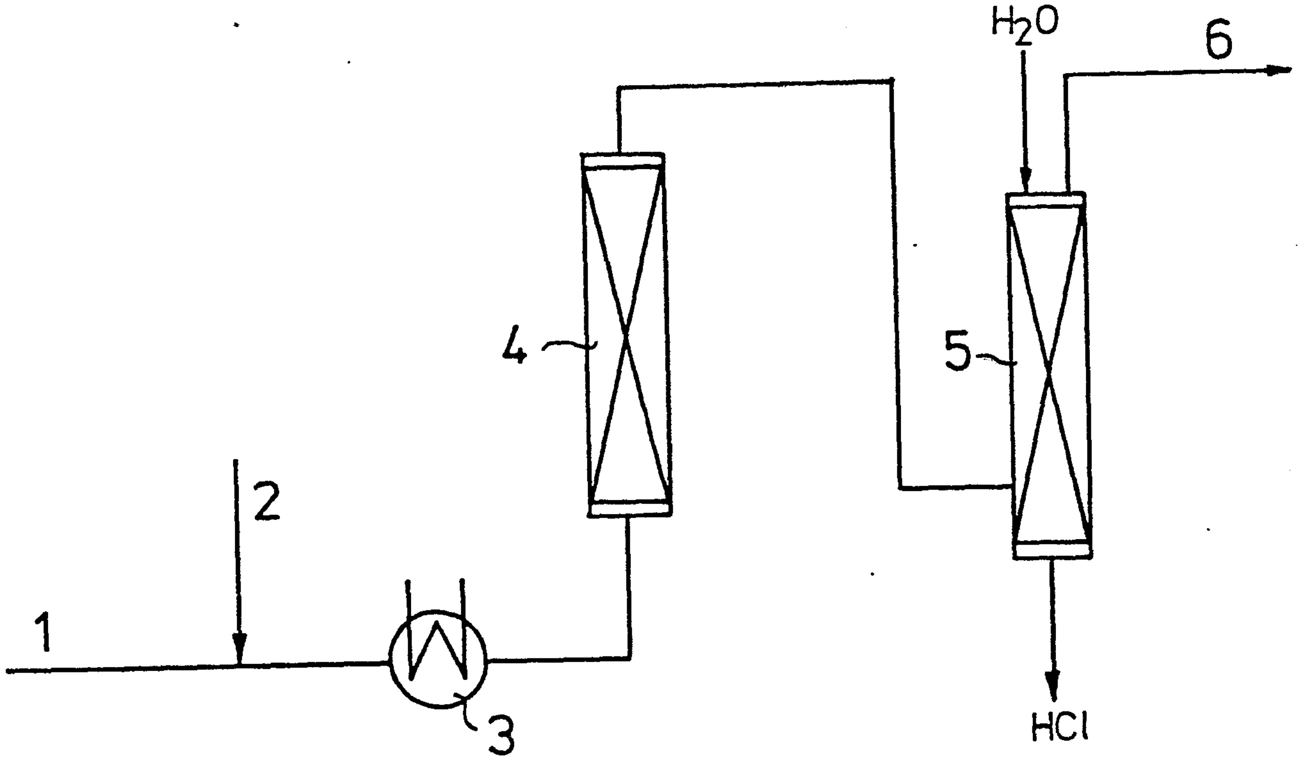
H2O
6
4
5
2
1
3
HCl

0199066

Europäisches Patentamt

# EUROPÄISCHER RECHERCHENBERICHT

Nummer der Anmeldung

EP 86 10 3447

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| X | BE-A- 525 596 (SOCIETE D'ELECTRO-CHIMIE, D'ELECTRO-METALLURGIE ET DES ACIERIES ELECTRIQUES D'UGINE) * Seite 4 * --- | 1-2,6-7,9,12-13 | C 07 C 1/26<br>C 07 C 17/00<br>C 07 B 35/06 |
| A | DE-B-1 057 080 (C.H. BOEHRINGER SOHN) --- | | |
| A | US-A-3 260 761 (H.O. BURRUS et al.) --- | | |
| A | US-A-4 155 941 (NYCHKA et al.) ----- | | |
| | | | RECHERCHIERTE SACHGEBIETE (Int Cl.4) |
| | | | C 07 C 1/00<br>C 07 C 17/00<br>C 07 B 35/00 |

Der vorliegende Recherchenbericht wurde für alle Patentansprüche erstellt

| Recherchenort | Abschlußdatum der Recherche | Prüfer |
|---|---|---|
| DEN HAAG | 16-07-1986 | VAN GEYT J.J.A. |

KATEGORIE DER GENANNTEN DOKUMENTE

X von besonderer Bedeutung allein betrachtet
Y von besonderer Bedeutung in Verbindung mit einer anderen Veroffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82